# EUROPEAN PATENT APPLICATION

(11) **EP 4 327 676 A1**
(43) Date of publication of application: **28.02.2024**
(21) Application number: 21948262.7
(22) Date of filing: 28.06.2021
(51) Int. Cl.: A24F 40/46

(54) **AEROSOL GENERATION SYSTEM**

(71) Applicant: Japan Tobacco, Inc., Tokyo, 105-6927 (JP)
(72) Inventor: YAMADA, Manabu, Tokyo 130-8603 (JP); INOUE, Yasunobu, Tokyo 130-8603 (JP)
(74) Representative: Hoffmann Eitle
(86) International application number: PCT/JP2021/024408
(87) International publication number: WO 2023/275950

(57) **Abstract**

[Problem] To provide an aerosol generation system having a heating unit that is more unlikely to break.

[Solution] This aerosol generation system comprises: an elongated heat generation unit that, when energized, generates heat to heat an aerosol generating substrate from the inside thereof; and a pair of metal plates disposed to respectively cover the facing surfaces of the heating unit along the elongated shape. The electrical resistance value of the heating unit in the direction in which the pair of metal plates faces each other is 100 to 1400 times the electrical resistance value of each of the pair of metal plates in the longitudinal direction of the elongated shape.

## Description

### Technical Field

The present invention relates to aerosol generation systems.

### Background Art

Inhaler devices including electronic cigarettes and nebulizers that generate material to be inhaled by users are becoming widely popular. Such an inhaler device uses an aerosol source for generating an aerosol and a flavor source for imparting a flavor component to the generated aerosol, so as to be capable of generating a flavor-component-imparted aerosol. A user can taste the flavor by inhaling the flavor-component-imparted aerosol generated by the inhaler device. The act of the user inhaling the aerosol may also be referred to as "puff" or "puff action" hereinafter.

In recent years, technology related to an inhaler device of a type that uses a stick-shaped substrate as an aerosol source or a flavor source is being actively developed. For example, Patent Literature 1 indicated below discloses a blade-shaped heater that is inserted into the stick-shaped substrate to heat the substrate from the inside thereof.

### Citation List

### Patent Literature

Patent Literature 1: CN 209807157 U

### Summary of Invention

### Technical Problem

Patent Literature 1 indicated above discloses that a ceramic heater is sandwiched between metallic electrodes and that electric current is applied to the ceramic heater via the electrodes, so that the ceramic heater is increased in temperature. However, in this configuration, the ceramic heater is increased in temperature non-uniformly, thus possibly making it difficult to achieve favorable aerosol generation.

The present invention has been made in view of the above problem, and an object of the present invention is to provide a mechanism that allows for more favorable aerosol generation.

### Solution to Problem

In order to solve the above problem, an aspect of the present invention provides an aerosol generation system including: a heat generator having a long shape, the heat generator generating heat by being supplied with electricity so as to heat an aerosol generating substrate from an inside thereof; and a pair of metal plates provided to respectively cover opposing surfaces of the heat generator along the long shape. An electrical resistance value of the heat generator in a direction in which the pair of metal plates face each other is 100 times to 1400 times an electrical resistance value of each of the pair of metal plates in a longitudinal direction of the long shape.

At a trailing end opposite a leading end to be inserted into the aerosol generating substrate, each of the pair of metal plates may be connected to an electric wire and be supplied with electricity.

The heat generator may be supplied with the electricity between the pair of metal plates.

The aerosol generation system may further include the aerosol generating substrate into which the heat generator covered by the pair of metal plates is inserted.

At least one of the pair of metal plates may include a rib formed by bending at least one of edges, in a lateral direction of the long shape, along the heat generator

A length of each of the pair of metal plates in the longitudinal direction of the long shape may be greater than a length of the heat generator

At a trailing end opposite a leading end to be inserted into the aerosol generating substrate, the pair of metal plates may be provided to extend further in the longitudinal direction relative to the heat generator.

The rib may be provided to extend entirely in the longitudinal direction of the heat generator.

The heat generator at a leading end to be inserted into the aerosol generating substrate may have an angularly protruding shape at the leading end.

At least one of the pair of metal plates may further include a leading-end rib formed by bending an edge along the shape at the leading end of the heat generator

A leading end of the heat generator to be inserted into the aerosol generating substrate may further be provided with a protrusion having an angularly protruding shape at the leading end.

The rib may be provided at each of opposite sides in the lateral direction of at least one of the pair of metal plates.

The rib may be provided at each of opposite sides in the lateral direction of both of the pair of metal plates.

The heat generator may have a tabular shape. A thickness of the tabular shape may be smaller than 1/4 of a width of the tabular shape.

The pair of metal plates may be provided at opposite principal surfaces of the tabular shape of the heat generator.

The heat generator and the pair of metal plates may be adhered together by using a conductive adhesive paste.

The pair of metal plates may be composed of a nickel-containing iron alloy.

The heat generator may be a PTC heater.

The PTC heater may include barium titanate.

A temperature of the heat generated by the heat generator may be below 350°C.

### Advantageous Effects of Invention

As described above, the present invention can provide a mechanism that allows for more favorable aerosol generation.

### Brief Description of Drawings

[FIG. 1] FIG. 1 is a schematic diagram schematically illustrating a configuration example of an inhaler device.
[FIG. 2] FIG. 2 is an exploded perspective view of a heater according to an embodiment of the present invention.
[FIG. 3] FIG. 3 is a top view of the heater illustrated in FIG. 2.
[FIG. 4] FIG. 4 is an exploded perspective view of a heater according to a first modification.
[FIG. 5] FIG. 5 is a top view of the heater illustrated in FIG. 5.
[FIG. 6] FIG. 6 is an exploded perspective view of a heater according to a second modification.
[FIG. 7] FIG. 7 is an exploded perspective view of a heater according to a third modification.
[FIG. 8] FIG. 8 is an exploded perspective view of a heater according to a fourth modification.

### Description of Embodiments

A preferred embodiment of the present invention will be described in detail below with reference to the appended drawings. In this description and the drawings, structural elements having substantially identical functional configurations will be given the same reference signs, and redundant descriptions thereof will be omitted.

### 1. Configuration example of inhaler device

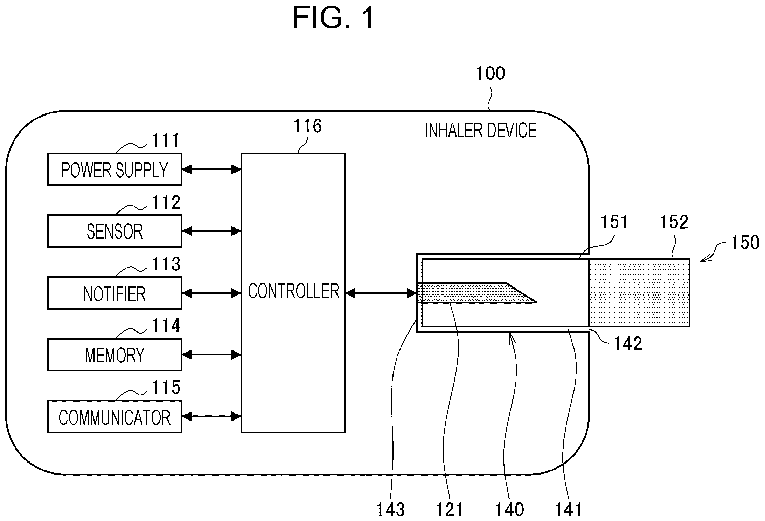

An inhaler device according to this configuration example generates an aerosol by heating a substrate containing an aerosol source from inside the substrate. A present configuration example will be described below with reference to FIG. 1.

FIG. 1 is a schematic diagram schematically illustrating a configuration example of the inhaler device. As illustrated in FIG. 1, an inhaler device 100 according to this configuration example includes a power supply 111, a sensor 112, a notifier 113, a memory 114, a communicator 115, a controller 116, a heater 121, and a container 140. Inhalation is performed by a user in a state where a stick substrate 150 is accommodated in the container 140. Each structural element will be sequentially described below.

The power supply 111 stores electric power. The power supply 111 supplies the electric power to the structural elements of the inhaler device 100. For example, the power supply 111 may be a rechargeable battery, such as a lithium ion secondary battery. The power supply 111 may be recharged by being connected to an external power supply by, for example, a USB (universal serial bus) cable. Alternatively, the power supply 111 may be recharged in a non-connected state with a power-transmitting device by wireless power transmission technology. As another alternative, the power supply 111 may be removable from the inhaler device 100 so as to be replaceable with a new power supply 111.

The sensor 112 detects various types of information regarding the inhaler device 100, and outputs the detected information to the controller 116. In an example, the sensor 112 may be a pressure sensor such as a microphone condenser, a flow sensor, or a temperature sensor. In such a case, when detecting a numerical value generated in accordance with the user's inhalation, the sensor 112 can output information indicating that the inhalation has been performed by the user to the controller 116. In another example, the sensor 112 is an input device, such as a button or a switch, receiving information input by the user. In particular, the sensor 112 may include a command button for starting/stopping aerosol generation. In such a case, the sensor 112 can output the information input by the user to the controller 116. In another example, the sensor 112 is a temperature sensor that detects the temperature of the heater 121. For example, the temperature sensor detects the temperature of the heater 121 based on an electrical resistance value of the heater 121. In such a case, the sensor 112 can detect the temperature of the stick substrate 150 accommodated in the container 140 based on the temperature of the heater 121.

The notifier 113 notifies the user of information. In an example, the notifier 113 is a light-emitting device, such as an LED (light-emitting diode). Accordingly, when the power supply 111 needs to be recharged, when the power supply 111 is being recharged, or when an abnormality has occurred in the inhaler device 100, the notifier 113 can emit light in different patterns of light, respectively. Each pattern of light is a concept involving colors and on/off timings. Together with or in place of the light-emitting device, the notifier 113 may be, for example, a display device that displays an image, a sound output device that outputs sound, and a vibration device that vibrates. The notifier 113 may also provide notification information indicating that inhalation by the user is possible. The notification information indicating that inhalation by the user is possible may be provided when the temperature of the stick substrate 150 heated by the heater 121 reaches a predetermined temperature.

The memory 114 stores various types of information for operation of the inhaler device 100. The memory 114 is, for example, a non-volatile storage medium, such as a flash memory. An example of the information stored in the memory 114 is information regarding the OS (operating system) of the inhaler device 100, such as the control contents of the various types of structural elements controlled by the controller 116. Another example of the information stored in the memory 114 is information regarding inhalation by the user, such as the number of times of inhalation, the inhalation time, and the accumulated inhalation time period.

The communicator 115 is a communication interface for exchanging information between the inhaler device 100 and another device. The communicator 115 performs communication in conformity with any wired or wireless communication standard. Such a communication standard may be, for example, a wireless LAN (local area network), a wired LAN, Wi-Fi (registered trademark), or Bluetooth (registered trademark). In an example, the communicator 115 transmits the information regarding the inhalation by the user to a smartphone to cause the smartphone to display the information regarding the inhalation by the user. In another example, the communicator 115 receives information about a new OS from a server to update the information about the OS stored in the memory 114.

The controller 116 functions as an arithmetic processing unit and a control device, and controls the overall operation in the inhaler device 100 in accordance with various programs. For example, the controller 116 is implemented by an electronic circuit, such as a CPU (central processing unit) or a microprocessor. Furthermore, the controller 116 may include a ROM (read only memory) that stores a program and arithmetic parameter to be used, and a RAM (random access memory) that temporarily stores an appropriately changing parameter. The inhaler device 100 executes various processes based on control by the controller 116. Examples of the processes controlled by the controller 116 include supplying of electric power from the power supply 111 to the other structural elements, recharging of the power supply 111, detection of information by the sensor 112, notification of information by the notifier 113, storing and reading of information by the memory 114, and exchanging of information by the communicator 115. Other processes executed by the inhaler device 100, such as input of information to each structural element and a process based on information output from each structural element, are also controlled by the controller 116.

The container 140 has an internal space 141 and holds the stick substrate 150 while accommodating a portion of the stick substrate 150 within the internal space 141. The container 140 has an opening 142 through which the internal space 141 communicates with the outside, and holds the stick substrate 150 inserted in the internal space 141 through the opening 142. For example, the container 140 is a tubular body having the opening 142 and a bottom 143 as a bottom surface, and defines the internal space 141 that is pillar-shaped. The container 140 has an inside diameter smaller than an outside diameter of the stick substrate 150 in at least a portion of the tubular body in the height direction, and may hold the stick substrate 150 while applying pressure around the stick substrate 150 inserted in the internal space 141. The container 140 also has a function for defining a flow path for air traveling through the stick substrate 150. An air inlet serving as an inlet for the air entering the flow path is disposed in, for example, the bottom 143. On the other hand, an air outlet serving as an outlet for the air exiting from the flow path is the opening 142.

The stick substrate 150 is a stick-shaped aerosol generating substrate. The stick substrate 150 includes a substrate 151 and an inhalation port 152.

The substrate 151 contains an aerosol source. The aerosol source atomizes by being heated, so that an aerosol is generated. The aerosol source may include, for example, a material derived from tobacco, such as a product obtained by forming shredded tobacco or tobacco raw material into a granular form, a sheet form, or a powder form. The aerosol source may also include a material not derived from tobacco and made from a plant (such as mint or herb) other than tobacco. If the inhaler device 100 is a medical inhaler, the aerosol source may include a medicine to be inhaled by a patient. The aerosol source is not limited to a solid and may be a liquid, such as polyhydric alcohol including glycerine and propylene glycol, or water. At least a portion of the substrate 151 is accommodated in the internal space 141 of the container 140 in the state where the stick substrate 150 is held by the container 140.

The inhalation port 152 is a member to be held in the user's mouth during inhalation. At least a portion of the inhalation port 152 protrudes from the opening 142 in the state where the stick substrate 150 is held by the container 140. When the user holds the inhalation port 152 protruding from the opening 142 in the user's mouth and inhales, air flows into the container 140 through the air inlet (not illustrated). The air flowing in travels through the internal space 141 of the container 140, that is, through the substrate 151, and reaches the inside of the user's mouth together with the aerosol generated from the substrate 151.

The heater 121 heats the aerosol source so as to atomize the aerosol source and generate the aerosol. As will be described in detail later, for example, the heater 121 is blade-shaped and is disposed to protrude from the bottom 143 of the container 140 to the internal space 141 of the container 140. Therefore, when the stick substrate 150 is inserted into the container 140, the blade-shaped heater 121 is inserted into the stick substrate 150 to pierce the substrate 151 of the stick substrate 150. When the heater 121 produces heat, the aerosol source contained in the stick substrate 150 atomizes by being heated from inside the stick substrate 150, whereby the aerosol is generated. The heater 121 produces heat when supplied with electric power from the power supply 111. In an example, when the sensor 112 detects that a predetermined user input has been performed, the aerosol may be generated by the heater 121 supplied with the electric power. When the temperature of the stick substrate 150 heated by the heater 121 reaches the predetermined temperature, inhalation by the user becomes possible. Subsequently, when the sensor 112 detects that a predetermined user input has been performed, the supply of electric power to the heater 121 may be stopped. In another example, in a time period in which the sensor 112 detects that the inhalation has been performed by the user, the aerosol may be generated by the heater 121 supplied with the electric power

The inhaler device 100 and the stick substrate 150 work in cooperation with each other to generate the aerosol to be inhaled by the user. Therefore, the combination of the inhaler device 100 and the stick substrate 150 may be regarded as an aerosol generation system.

### 2. Detailed configuration of heater

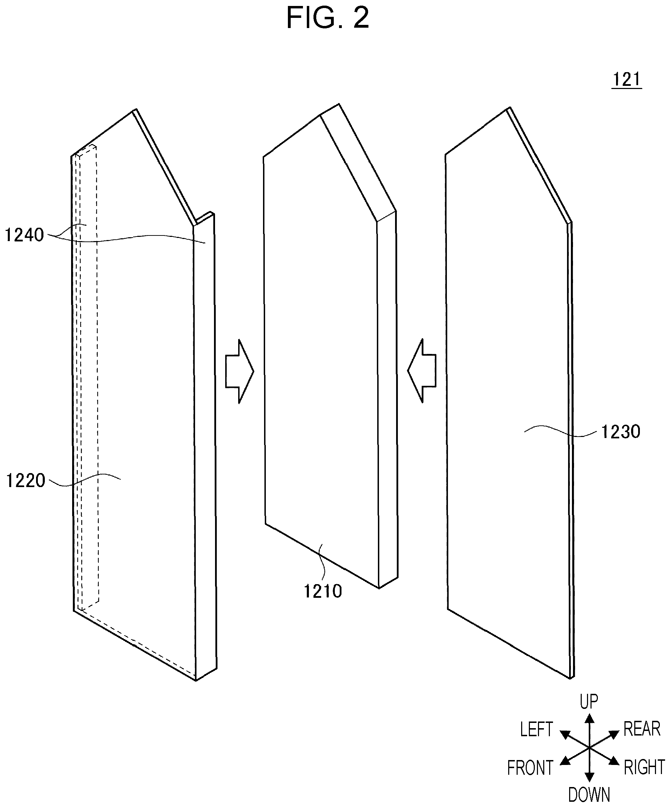

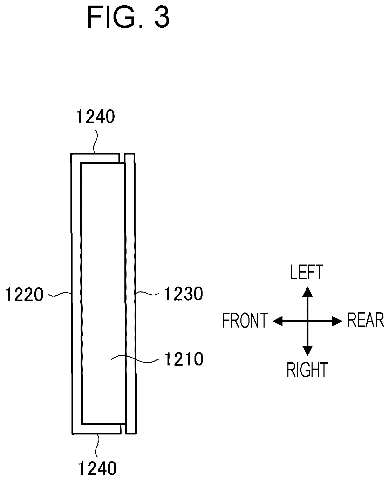

Next, the heater 121 included in the inhaler device 100 according to this embodiment will be described in further detail with reference to FIG. 2 and FIG. 3. FIG. 2 is an exploded perspective view of the heater 121 according to this embodiment. FIG. 3 is a top view of the heater 121 illustrated in FIG. 2.

As illustrated in FIG. 2 and FIG. 3, the heater 121 includes the heat generator 1210, a first metal plate 1220, and a second metal plate 1230. The heater 121 heats the stick substrate 150 from the inside thereof by using heat generated by the heat generator 1210 supplied with electricity via the first metal plate 1220 and the second metal plate 1230.

In FIG. 2 and FIG. 3, a direction in which the leading end of the heater 121 is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

The heat generator 1210 is a long-shaped member that generates heat by resistance heating. In detail, the heat generator 1210 may be a PTC (positive temperature coefficient) heater that generates heat when electricity is supplied between the first metal plate 1220 and the second metal plate 1230.

A PTC heater uses a resistor having properties (PTC properties) in which an electrical resistance value increases significantly when the temperature reaches a predetermined temperature (referred to as "Curie temperature") such that an electric current does not flow therethrough. By utilizing the PTC properties, a PTC heater can control the amount of supplied electricity without having to use a control device, so as to be capable of controlling the heating temperature below the Curie temperature. Therefore, a PTC heater can heat a target below the Curie temperature. For example, the heat generator 1210 may be a PTC heater with barium titanate (BaTiO₃) having the PTC properties as the resistor. In such a case, the heat generator 1210 can set the Curie temperature of the barium titanate to 350°C, so as to be capable of heating the stick substrate 150 to a temperature below 350°C.

The heat generator 1210 may have a long tabular shape extending in the up-down direction. Specifically, the longitudinal direction of the long shape of the heat generator 1210 corresponds to the up-down direction, whereas the lateral direction of the long shape corresponds to the left-right direction. By having the long tabular shape, the heat generator 1210 has a rectangular cross-sectional shape that is orthogonal to the longitudinal direction (i.e., the up-down direction) of the long shape, as illustrated in FIG. 3. Accordingly, as compared with a case where the heat generator 1210 has a circular cross-sectional shape, the cross-sectional shape can have a longer perimeter even with the same cross-sectional area. Therefore, the heat generator 1210 can allow for a larger contact area between the heater 121 and the stick substrate 150 to which the heater 121 is to be inserted, whereby the stick substrate 150 can be heated more efficiently. For example, the tabular shape of the heat generator 1210 may have a thickness smaller than 1/4 of the width of the long shape in the lateral direction (i.e., the left-right direction).

The heat generator 1210 at the leading end to be inserted into the stick substrate 150 may have an angularly protruding shape toward the leading end (i.e., in the up direction). The angular shape extending toward the leading end may have an acute angle, a right angle, or an obtuse angle. For example, the heat generator 1210 may have a pentagonal tabular shape whose apex exists at the leading end (i.e., the upper end) to be inserted into the stick substrate 150 and that extends in the up-down direction. With regard to the heat generator 1210, the leading end (i.e., the upper end) thereof to be inserted into the stick substrate 150 has a pointy shape like a sword tip, so that the heater 121 can be inserted into the stick substrate 150 more readily.

The first metal plate 1220 and the second metal plate 1230 are a pair of electrode plates sandwiching the heat generator 1210 therebetween. In detail, the first metal plate 1220 and the second metal plate 1230 may be provided at opposite principal surfaces opposing each other in the front-rear direction of the tabular heat generator 1210. The first metal plate 1220 and the second metal plate 1230 are provided apart from each other to prevent a short-circuit.

The first metal plate 1220 and the second metal plate 1230 are bonded to the heat generator 1210 by using a conductive adhesive paste, so that electricity can be supplied to the heat generator 1210. An example of the conductive adhesive paste that can be used is a so-called anisotropic conductive adhesive having conductive particles uniformly distributed within an epoxy-based adhesive.

The first metal plate 1220 and the second metal plate 1230 may be composed of metal with a low thermal expansion coefficient. For example, the first metal plate 1220 and the second metal plate 1230 may be composed of a nickel (Ni) containing iron alloy with a low thermal expansion coefficient, such as Invar (registered trademark). Accordingly, delamination of the first metal plate 1220 and the second metal plate 1230 from the heat generator 1210 due to thermal expansion occurring when the heat generator 1210 generates heat can be suppressed.

The first metal plate 1220 and the second metal plate 1230 may be provided to cover the heat generator 1210 by having a shape that conforms with the shape of the heat generator 1210. In detail, the first metal plate 1220 and the second metal plate 1230 may each have a shape that extends further in the longitudinal direction (i.e., the up-down direction) relative to the long shape of the heat generator 1210. The first metal plate 1220 and the second metal plate 1230 at the leading end to be inserted into the stick substrate 150 may each have an angularly protruding shape toward the leading end (i.e., in the up direction). The angular shape extending toward the leading end may have an acute angle, a right angle, or an obtuse angle. For example, the first metal plate 1220 and the second metal plate 1230 may each be similar to the heat generator 1210 in having a pentagonal tabular shape whose apex exists at the leading end (i.e., the upper end) to be inserted into the stick substrate 150 and that extends in the up-down direction. With regard to each of the first metal plate 1220 and the second metal plate 1230, the leading end (i.e., the upper end) thereof to be inserted into the stick substrate 150 has a pointy shape like a sword tip, so that the heater 121 can be inserted into the stick substrate 150 more readily. The first metal plate 1220 and the second metal plate 1230 may have the same shape or may have shapes different from each other.

Each of the first metal plate 1220 and the second metal plate 1230 at the trailing end (i.e., the lower end) opposite the leading end may extend further downward relative to the trailing end of the heat generator 1210. The first metal plate 1220 and the second metal plate 1230 in regions extending further downward relative to the trailing end of the heat generator 1210 may be provided with, for example, a securing section. The securing section (not illustrated) is a structural member that secures the heater 121 to a housing of the inhaler device 100. By holding the heater 121 at a region located away from the heat generator 1210, the securing section is less likely to be affected by the heat generated by the heat generator 1210, so that the material used for forming the securing section can be selected more flexibly.

At the trailing end (i.e., the lower end), each of the first metal plate 1220 and the second metal plate 1230 is connected to an electric wire and is supplied with electricity. For example, the lower end of the first metal plate 1220 and the lower end of the second metal plate 1230 are connected to electric wires and are supplied with electricity. As a result, electric current flowing in from the lower end of the first metal plate 1220 or the second metal plate 1230 travels through the heat generator 1210 and flows out from the lower end of the other first metal plate 1220 or second metal plate 1230.

An electrical resistance value of the heat generator 1210 in a direction (i.e., the front-rear direction) in which the first metal plate 1220 and the second metal plate 1230 face each other is desirably 100 times to 1400 times an electrical resistance value of each of the first metal plate 1220 and the second metal plate 1230 in the longitudinal direction (i.e., the up-down direction) of the long shape of the heat generator 1210. In particular, the electrical resistance value in the front-rear direction of the heat generator 1210 is desirably 100 times to 1400 times the electrical resistance value in the up-down direction of an area of each of the first metal plate 1220 and the second metal plate 1230 in contact with the heat generator 1210. As an example, the electrical resistance value of the heat generator 1210 in the front-rear direction may be between 1.0 and 1.4 [Ω], and the electrical resistance value of each of the first metal plate 1220 and the second metal plate 1230 in the up-down direction may be between 0.001 and 0.01 [Ω].

Accordingly, the electrical resistance value of the heat generator 1210 in the front-rear direction is set to be significantly higher than the electrical resistance value of each of the first metal plate 1220 and the second metal plate 1230 in the up-down direction, thereby allowing for favorable aerosol generation. Specifically, in the first metal plate 1220 or the second metal plate 1230, the electric current flowing in from the lower end spreads entirely in the up-down direction, so that the heat generator 1210 can be supplied with electricity uniformly in the up-down direction. In other words, the electric current can be prevented from flowing downward in a concentrated manner through the heat generator 1210. As a result, the heat generator 1210 can generate heat uniformly in the up-down direction, so that the stick substrate 150 can be heated uniformly. Accordingly, this allows for favorable aerosol generation.

As a comparative example for causing the heat generator 1210 to generate heat uniformly in the up-down direction, it is conceivable to set the electrical resistance value of the heat generator 1210 in the front-rear direction to decrease in the up direction and to increase in the down direction. As an example, a conceivable comparative example is to provide the thickness (i.e., the length in the front-rear direction) of the heat generator 1210 or the density of the heat generator 1210 with a difference in the up-down direction. However, in order to realize this configuration, an additional process for machining the heat generator 1210 is required. In this regard, this embodiment can eliminate such an additional process required in the comparative example.

In the heater 121 included in the inhaler device 100 according to this embodiment, at least one of the first metal plate 1220 and the second metal plate 1230 is further provided with ribs 1240.

In detail, as illustrated in FIG. 2 and FIG. 3, the ribs 1240 may be formed by bending opposite edges, in the lateral direction (i.e., the left-right direction) of the long shape of the first metal plate 1220, entirely along the outer shape of the heat generator 1210. For example, if the first metal plate 1220 has a pentagonal shape extending in the up-down direction, the ribs 1240 may be formed by entirely bending the opposite left and right edges extending from the first metal plate 1220.

With the ribs 1240 provided, the first metal plate 1220 has increased strength in the front-rear direction (i.e., the normal direction to the principal surfaces of the first metal plate 1220) in which the ribs 1240 are bent, so that deformation in the normal direction can be suppressed. Accordingly, the heater 121 is less likely to deform in the normal direction (i.e., the front-rear direction) to the principal surfaces of the first metal plate 1220, so that the possibility of breakage of the heater 121 in the normal direction can be reduced.

With the above configuration, for example, the heater 121 according to this embodiment can have increased strength in the normal direction (i.e., the front-rear direction) to the principal surfaces of the heater 121 in accordance with the ribs 1240 provided at the first metal plate 1220. Therefore, the heater 121 according to this embodiment can have increased strength in the front-rear direction that is relatively lower than the strength in the up-down direction and the left-right direction, so that the possibility of breakage of the heater 121 when inserted into the stick substrate 150 can be reduced.

### 3. Modifications

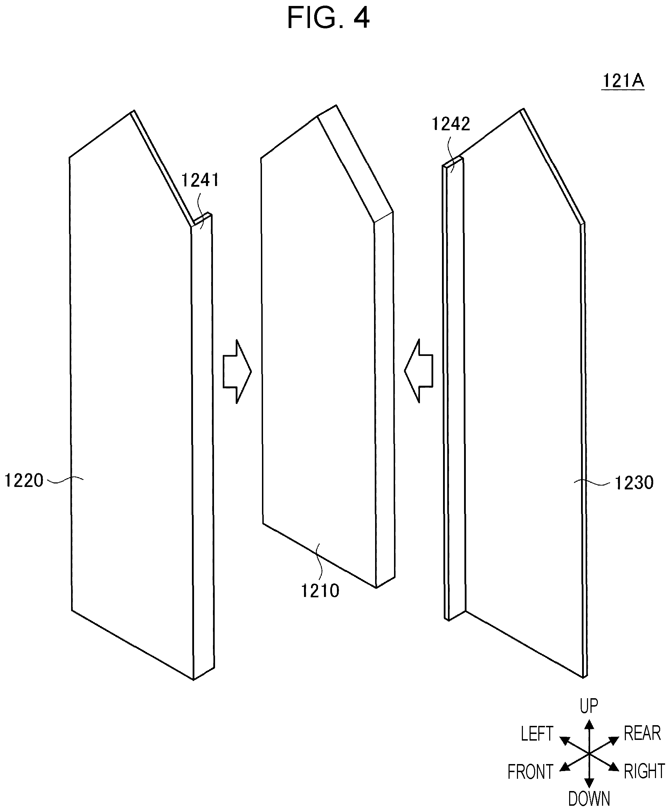

First to fifth modifications of the heater 121 according to this embodiment will now be described with reference to FIG. 4 to FIG. 8.

### (First Modification)

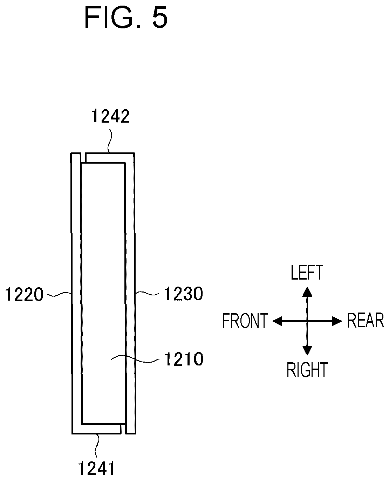

FIG. 4 is an exploded perspective view of a heater 121A according to a first modification. FIG. 5 is a top view of the heater 121A illustrated in FIG. 4.

In FIG. 4 and FIG. 5, the up-down direction, the front-rear direction, and the left-right direction are defined similarly to FIG. 2 and FIG. 3. In detail, a direction in which the leading end of the heater 121A is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 4 and FIG. 5, in the heater 121A according to the first modification, the first metal plate 1220 is provided with a first rib 1241, and the second metal plate 1230 is provided with a second rib 1242.

In detail, the first rib 1241 may be formed by bending one of the edges, in the lateral direction (i.e., the left-right direction) of the long shape of the first metal plate 1220, along the outer shape of the heat generator 1210. The second rib 1242 may be formed by bending the other one of the edges, in the lateral direction (i.e., the left-right direction) of the long shape of the second metal plate 1230, along the outer shape of the heat generator 1210. For example, if the first metal plate 1220 and the second metal plate 1230 have a pentagonal shape extending in the up-down direction, the first rib 1241 may be formed by bending the right edge extending from the first metal plate 1220. Moreover, the second rib 1242 may be formed by bending the left edge extending from the second metal plate 1230.

With the first rib 1241 and the second rib 1242 provided, the first metal plate 1220 and the second metal plate 1230 have increased strength in the front-rear direction in which the first rib 1241 and the second rib 1242 are bent, so that deformation in the normal direction can be suppressed. Accordingly, the heater 121A is less likely to deform in the normal direction (i.e., the front-rear direction) to the principal surfaces of the first metal plate 1220 and the second metal plate 1230, so that the possibility of breakage of the heater 121A in the normal direction can be reduced.

Specifically, the first rib 1241 and the second rib 1242 may be provided at both of the pair of electrode plates (i.e., the first metal plate 1220 and the second metal plate 1230). In such a case, the heater 121A according to the first modification is similar to the heater 121 provided with the ribs 1240 only at the first metal plate 1220 in that the possibility of breakage of the heater 121A when inserted into the stick substrate 150 can be reduced.

### (Second Modification)

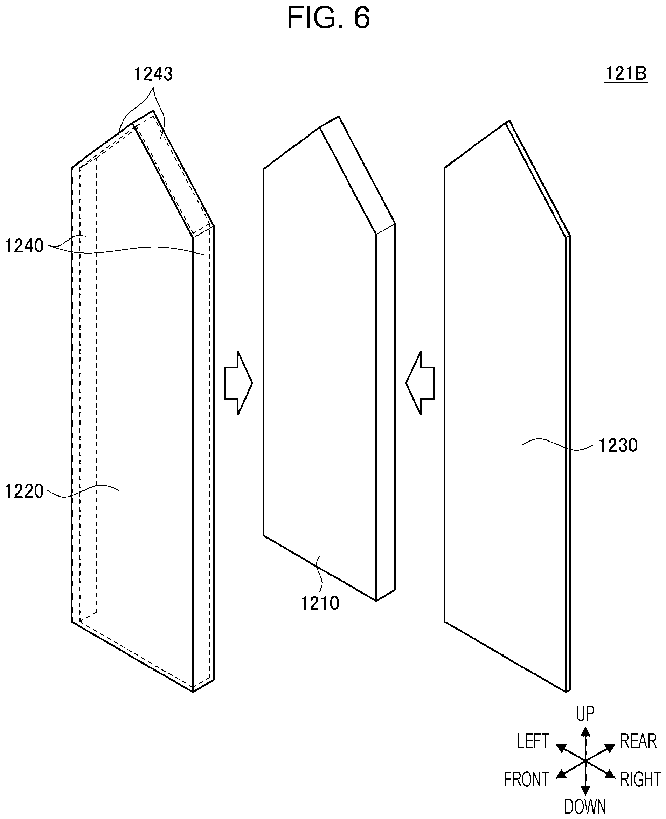

FIG. 6 is an exploded perspective view of a heater 121B according to a second modification.

In FIG. 6, the up-down direction, the front-rear direction, and the left-right direction are defined similarly to FIG. 2. In detail, a direction in which the leading end of the heater 121B is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 6, in addition to the ribs 1240, the heater 121B according to the second modification is further provided with leading-end ribs 1243 in conformity with the angularly protruding shape toward the leading end (i.e., in the up direction) of the heat generator 1210.

In detail, the leading-end ribs 1243 may be formed by bending upper edges (located toward the leading end of the heat generator 1210) of the first metal plate 1220 along the outer shape of the heat generator 1210. For example, if the first metal plate 1220 has a pentagonal shape extending in the up-down direction, the leading-end ribs 1243 may be formed by bending two upper edges of the first metal plate 1220. In such a case, the first metal plate 1220 has the ribs 1240 and the leading-end ribs 1243 at four edges excluding the lower edge of the pentagonal shape.

With the leading-end ribs 1243 provided, the first metal plate 1220 can cover the sword-tip-like pointy-shaped leading end (i.e., the upper end) of the heat generator 1210 with the leading-end ribs 1243. Accordingly, when inserted into the stick substrate 150, the heater 121B can prevent delamination of the first metal plate 1220 and the second metal plate 1230 from the heat generator 1210 due to stress acting among the heat generator 1210, the first metal plate 1220, and the second metal plate 1230. Therefore, the heater 121B can further improve the durability with respect to insertion thereof into the stick substrate 150.

### (Third Modification)

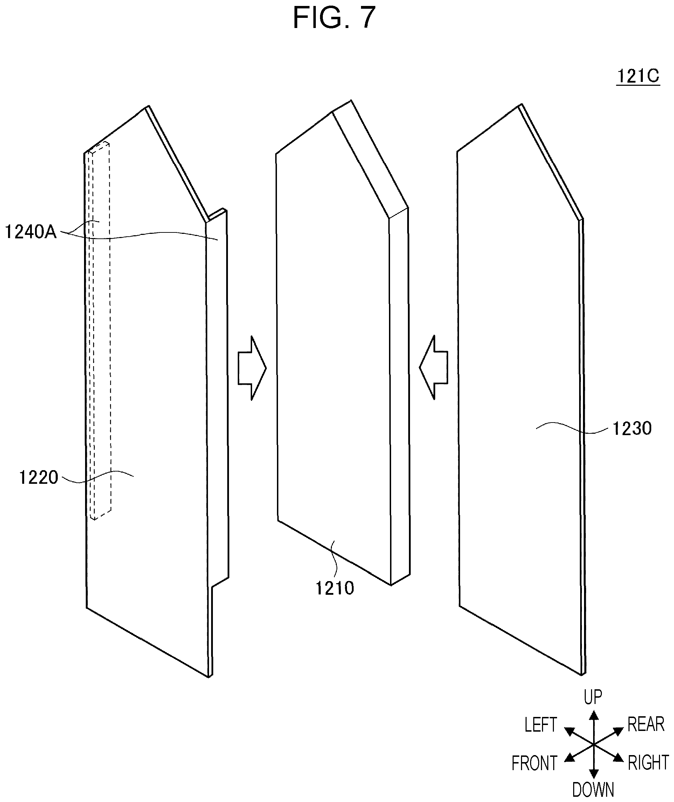

FIG. 7 is an exploded perspective view of a heater 121C according to a third modification.

In FIG. 7, the up-down direction, the front-rear direction, and the left-right direction are defined similarly to FIG. 2. In detail, a direction in which the leading end of the heater 121C is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 7, in the heater 121C according to the third modification, at least one of the first metal plate 1220 and the second metal plate 1230 is provided with ribs 1240A.

In detail, the ribs 1240A may be formed by bending opposite edges, in the lateral direction (i.e., the left-right direction) of the long shape of the first metal plate 1220, partially along the outer shape of the heat generator 1210. For example, if the first metal plate 1220 has a pentagonal shape extending in the up-down direction, the ribs 1240A may be formed by partially bending the opposite left and right edges extending from the first metal plate 1220.

Specifically, each rib 1240A may be provided in only a partial region located toward the leading end (i.e., the upper end) of the corresponding one of the left and right edges of the first metal plate 1220. In such a case, the ribs 1240A are provided at the regions of the first metal plate 1220 to be inserted into the stick substrate 150, so that the strength of the heater 121C at the regions to be inserted into the stick substrate 150 can be increased. Therefore, the heater 121C according to the third modification is similar to the heater 121 provided with the ribs 1240 entirely along the opposite left and right edges of the first metal plate 1220 in that the possibility of breakage of the heater 121C when inserted into the stick substrate 150 can be reduced.

### (Fourth Modification)

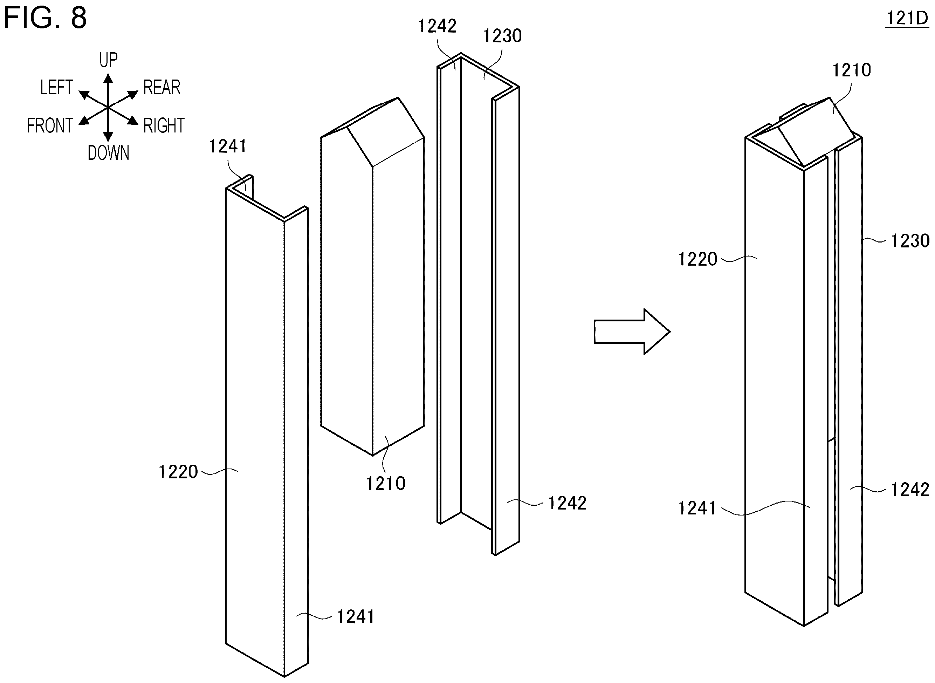

FIG. 8 is an exploded perspective view of a heater 121D according to a fourth modification.

In FIG. 8, the up-down direction, the front-rear direction, and the left-right direction are defined similarly to FIG. 2. In detail, a direction in which the leading end of the heater 121D is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 8, in the heater 121D according to the fourth modification, the first metal plate 1220 is provided with first ribs 1241, and the second metal plate 1230 is provided with second ribs 1242.

In detail, the first ribs 1241 may be formed by bending opposite edges, in the lateral direction (i.e., the left-right direction) of the long shape of the first metal plate 1220, along the outer shape of the heat generator 1210. The second ribs 1242 may be formed by bending opposite edges, in the lateral direction (i.e., the left-right direction) of the long shape of the second metal plate 1230, along the outer shape of the heat generator 1210. For example, if the first metal plate 1220 and the second metal plate 1230 have a pentagonal shape extending in the up-down direction, the first ribs 1241 may be formed by bending the opposite edges in the lateral direction of the first metal plate 1220. Moreover, the second ribs 1242 may be formed by bending the opposite edges in the lateral direction of the second metal plate 1230.

In such a case, the heat generator 1210 may have a shape with a larger thickness to prevent a short-circuit between the first ribs 1241 and the second ribs 1242. For example, the heat generator 1210 may have a prismatic shape extending in the up-down direction. The heat generator 1210 at the leading end to be inserted into the stick substrate 150 may protrude to form a ridge toward the leading end (i.e., in the up direction).

With the first ribs 1241 and the second ribs 1242 provided, the first metal plate 1220 and the second metal plate 1230 have increased strength in the front-rear direction in which the first ribs 1241 and the second ribs 1242 are bent, so that deformation in the normal direction can be suppressed. Accordingly, the heater 121D is less likely to deform in the normal direction (i.e., the front-rear direction) to the principal surfaces of the first metal plate 1220 and the second metal plate 1230, so that the possibility of breakage of the heater 121A in the normal direction can be reduced. Consequently, the heater 121D according to the fourth modification is similar to the heater 121 illustrated in FIG. 2 in that the possibility of breakage of the heater 121D when inserted into the stick substrate 150 can be reduced.

### (Fifth Modification)

In the heater 121 according to a fifth modification, the heat generator 1210, the first metal plate 1220, and the second metal plate 1230 are provided with a protrusion at the leading end (i.e., the upper end) thereof. In detail, the heat generator 1210, the first metal plate 1220, and the second metal plate 1230 each have a rectangular tabular shape extending in the up-down direction, and the protrusion is provided on the upper edges of the heat generator 1210, the first metal plate 1220, and the second metal plate 1230.

The protrusion is composed of a highly rigid material, such as a ceramic material or metal, and has a triangular or pentagonal tabular shape protruding angularly toward the leading end (i.e., in the up direction). Accordingly, with regard to the heater 121, the leading end (i.e., the upper end) thereof to be inserted into the stick substrate 150 can have a pointy shape like a sword tip without having to machine the heat generator 1210, the first metal plate 1220, and the second metal plate 1230 into a pentagonal shape. Consequently, the heater 121 can be inserted into the stick substrate 150 more readily. Furthermore, the thickness of the protrusion in the front-rear direction may be equal to the sum of the thicknesses of the heat generator 1210, the first metal plate 1220, and the second metal plate 1230, or may be equal to the thickness of the heat generator 1210.

When the heater 121 is inserted into the stick substrate 150, the protrusion can prevent stress from acting among the heat generator 1210, the first metal plate 1220, and the second metal plate 1230. Accordingly, the protrusion can prevent the first metal plate 1220 and the second metal plate 1230 from delaminating from the heat generator 1210, thereby further improving the durability of the heater 121 with respect to insertion thereof into the stick substrate 150.

Although a preferred embodiment of the present invention has been described in detail above with reference to the appended drawings, the present invention is not limited to this example. It is apparent to a person with a common knowledge of the technical field to which the present invention belongs that various modifications and alterations are conceivable within the scope of the technical ideas defined in the claims, and it is to be understood that such modifications and alterations naturally belong to the technical scope of the present invention.

For example, although the above embodiment relates to an example where the first metal plate 1220 and the second metal plate 1230 are bonded to the heat generator 1210 by using a conductive adhesive paste, the present invention is not limited to this example. As an example, each of the first metal plate 1220 and the second metal plate 1230 and the heat generator 1210 may have a thermal conduction layer composed of a highly thermally conductive material interposed therebetween and may be bonded together by using a conductive adhesive paste. As another example, the first metal plate 1220 and the second metal plate 1230 may be bonded to the heat generator 1210 by using a conductive adhesive paste mixed with a highly thermally conductive material. Examples of the highly thermally conductive material include silicon, SiC, and SiV With this configuration, the first metal plate 1220 and the second metal plate 1230 can be increased in temperature more efficiently.

For example, although this embodiment relates to an example where the heat generator 1210 at the leading end to be inserted into the stick substrate 150 has an angularly protruding shape toward the leading end (i.e., in the up direction), the present invention is not limited to this example. For example, the leading end of the heat generator 1210 may have a flat shape, and the leading end surface of the flat-shaped heat generator 1210 may be provided with a rigid body having an angularly protruding shape toward the leading end (i.e., in the up direction).

For example, although this embodiment relates to an example where the first metal plate 1220 and the second metal plate 1230 are composed of metal having a low coefficient of thermal expansion, the present invention is not limited to this example. For example, the first metal plate 1220 and the second metal plate 1230 may be composed of a material having a coefficient of thermal expansion close to the coefficient of thermal expansion of a ceramic material used for forming the heat generator 1210. An example of such a material is SUS 430. This configuration can similarly suppress delamination of the first metal plate 1220 and the second metal plate 1230 from the heat generator 1210 due to thermal expansion occurring when the heat generator 1210 generates heat.

The following configurations also belong to the technical scope of the present invention.
(1) An aerosol generation system comprising:
   a heat generator having a long shape, the heat generator generating heat by being supplied with electricity so as to heat an aerosol generating substrate from an inside thereof; and
   a pair of metal plates provided to respectively cover opposing surfaces of the heat generator along the long shape,
   wherein an electrical resistance value of the heat generator in a direction in which the pair of metal plates face each other is 100 times to 1400 times an electrical resistance value of each of the pair of metal plates in a longitudinal direction of the long shape.
(2) The aerosol generation system according to (1), wherein, at a trailing end opposite a leading end to be inserted into the aerosol generating substrate, each of the pair of metal plates is connected to an electric wire and is supplied with electricity.
(3) The aerosol generation system according to (2), wherein the heat generator is supplied with the electricity between the pair of metal plates.
(4) The aerosol generation system according to any one of (1) to (3), further comprising the aerosol generating substrate into which the heat generator covered by the pair of metal plates is inserted.
(5) The aerosol generation system according to any one of (1) to (4), wherein at least one of the pair of metal plates includes a rib formed by bending at least one of edges, in a lateral direction of the long shape, along the heat generator
(6) The aerosol generation system according to any one of (1) to (5), wherein a length of each of the pair of metal plates in the longitudinal direction of the long shape is greater than a length of the heat generator.
(7) The aerosol generation system according to (6), wherein, at a trailing end opposite a leading end to be inserted into the aerosol generating substrate, the pair of metal plates are provided to extend further in the longitudinal direction relative to the heat generator.
(8) The aerosol generation system according to (7) as directly or indirectly dependent on (5), wherein the rib is provided to extend entirely in the longitudinal direction of the heat generator.
(9) The aerosol generation system according to any one of (5) to (8), wherein the heat generator at a leading end to be inserted into the aerosol generating substrate has an angularly protruding shape at the leading end.
(10) The aerosol generation system according to (9), wherein at least one of the pair of metal plates further includes a leading-end rib formed by bending an edge along the shape at the leading end of the heat generator.
(11) The aerosol generation system according to any one of (1) to (8), wherein a leading end of the heat generator to be inserted into the aerosol generating substrate is further provided with a protrusion having an angularly protruding shape at the leading end.
(12) The aerosol generation system according to (5) or any one of (6) to (11) as directly or indirectly dependent on (5), wherein the rib is provided at each of opposite sides in the lateral direction of at least one of the pair of metal plates.
(13) The aerosol generation system according to (5) or any one of (6) to (12) as directly or indirectly dependent on (5), wherein the rib is provided at each of opposite sides in the lateral direction of both of the pair of metal plates.
(14) The aerosol generation system according to any one of (1) to (13),
   wherein the heat generator has a tabular shape, and
   wherein a thickness of the tabular shape is smaller than 1/4 of a width of the tabular shape.
(15) The aerosol generation system according to (14), wherein the pair of metal plates are provided at opposite principal surfaces of the tabular shape of the heat generator
(16) The aerosol generation system according to any one of (1) to (15), wherein the heat generator and the pair of metal plates are adhered together by using a conductive adhesive paste.
(17) The aerosol generation system according to any one of (1) to (16), wherein the pair of metal plates are composed of a nickel-containing iron alloy.
(18) The aerosol generation system according to any one of (1) to (17), wherein the heat generator is a PTC heater.
(19) The aerosol generation system according to (18), wherein the PTC heater includes barium titanate.
(20) The aerosol generation system according to (18) or (19), wherein a temperature of the heat generated by the heat generator is below 350°C.

### Reference Signs List

- 100: inhaler device
- 111: power supply
- 112: sensor
- 113: notifier
- 114: memory
- 115: communicator
- 116: controller
- 121, 121A, 121B, 121C, 121D: heater
- 140: container
- 141: internal space
- 142: opening
- 143: bottom
- 150: stick substrate
- 151: substrate
- 152: inhalation port
- 1210: heat generator
- 1220: first metal plate
- 1230: second metal plate
- 1240, 1240A: rib
- 1241: first rib
- 1242: second rib
- 1243: leading-end rib

## Claims

1. An aerosol generation system comprising:
a heat generator having a long shape, the heat generator generating heat by being supplied with electricity so as to heat an aerosol generating substrate from an inside thereof; and
a pair of metal plates provided to respectively cover opposing surfaces of the heat generator along the long shape,
wherein an electrical resistance value of the heat generator in a direction in which the pair of metal plates face each other is 100 times to 1400 times an electrical resistance value of each of the pair of metal plates in a longitudinal direction of the long shape.

2. The aerosol generation system according to claim 1, wherein, at a trailing end opposite a leading end to be inserted into the aerosol generating substrate, each of the pair of metal plates is connected to an electric wire and is supplied with electricity.

3. The aerosol generation system according to claim 2, wherein the heat generator is supplied with the electricity between the pair of metal plates.

4. The aerosol generation system according to any one of claims 1 to 3, further comprising the aerosol generating substrate into which the heat generator covered by the pair of metal plates is inserted.

5. The aerosol generation system according to any one of claims 1 to 4, wherein at least one of the pair of metal plates includes a rib formed by bending at least one of edges, in a lateral direction of the long shape, along the heat generator

6. The aerosol generation system according to any one of claims 1 to 5, wherein a length of each of the pair of metal plates in the longitudinal direction of the long shape is greater than a length of the heat generator.

7. The aerosol generation system according to claim 6, wherein, at a trailing end opposite a leading end to be inserted into the aerosol generating substrate, the pair of metal plates are provided to extend further in the longitudinal direction relative to the heat generator.

8. The aerosol generation system according to claim 7 as directly or indirectly dependent on claim 5, wherein the rib is provided to extend entirely in the longitudinal direction of the heat generator.

9. The aerosol generation system according to any one of claims 5 to 8, wherein the heat generator at a leading end to be inserted into the aerosol generating substrate has an angularly protruding shape at the leading end.

10. The aerosol generation system according to claim 9, wherein at least one of the pair of metal plates further includes a leading-end rib formed by bending an edge along the shape at the leading end of the heat generator.

11. The aerosol generation system according to any one of claims 1 to 8, wherein a leading end of the heat generator to be inserted into the aerosol generating substrate is further provided with a protrusion having an angularly protruding shape at the leading end.

12. The aerosol generation system according to claim 5 or any one of claims 6 to 11 as directly or indirectly dependent on claim 5, wherein the rib is provided at each of opposite sides in the lateral direction of at least one of the pair of metal plates.

13. The aerosol generation system according to claim 5 or any one of claims 6 to 12 as directly or indirectly dependent on claim 5, wherein the rib is provided at each of opposite sides in the lateral direction of both of the pair of metal plates.

14. The aerosol generation system according to any one of claims 1 to 13,
wherein the heat generator has a tabular shape, and
wherein a thickness of the tabular shape is smaller than 1/4 of a width of the tabular shape.

15. The aerosol generation system according to claim 14, wherein the pair of metal plates are provided at opposite principal surfaces of the tabular shape of the heat generator

16. The aerosol generation system according to any one of claims 1 to 15, wherein the heat generator and the pair of metal plates are adhered together by using a conductive adhesive paste.

17. The aerosol generation system according to any one of claims 1 to 16, wherein the pair of metal plates are composed of a nickel-containing iron alloy.

18. The aerosol generation system according to any one of claims 1 to 17, wherein the heat generator is a PTC heater.

19. The aerosol generation system according to claim 18, wherein the PTC heater includes barium titanate.

20. The aerosol generation system according to claim 18 or 19, wherein a temperature of the heat generated by the heat generator is below 350°C.
